# EUROPEAN PATENT SPECIFICATION

(11) **EP 2 614 142 B1**
(45) Date of publication and mention of the grant of the patent: **17.06.2015**
(21) Application number: 11767394.7
(22) Date of filing: 09.09.2011
(51) Int. Cl.: C12N 5/076, C12M 3/00, A61B 17/43

(54) **METHOD FOR ENRICHMENT OF DNA STRAND BREAK-FREE SPERMATOZOA AND REDUCTION OF RISKS FOR ABNORMALITIES AND/OR ANEUPLOIDY**
VERFAHREN ZUR ANREICHERUNG VON SPERMATOZOEN OHNE DNA-STRANGBRÜCHE UND VERRINGERUNG DES RISIKOS VON ANOMALIEN UND/ODER ANEUPLOIDIE
PROCÉDÉ D'ENRICHISSEMENT EN SPERMATOZOÏDES SANS CASSURE DES BRINS D'ADN ET RÉDUCTION DES RISQUES D'ANOMALIES ET/OU D'ANEUPLOÏDIE

(30) Priority: 09.09.2010 EP 10009406
(43) Date of publication of application: 17.07.2013
(73) Proprietor: ZECH, Josef, 6020 Innsbruck (AT)
(72) Inventor: ZECH, Josef, 6020 Innsbruck (AT)
(74) Representative: Vossius & Partner Patentanwälte Rechtsanwälte mbB
(86) International application number: PCT/EP2011/065665
(87) International publication number: WO 2012/032165

(56) References cited:
- WO-A2-03/031564
- "Der Zech Selektor - Innovation als Garant für den Erfolg bei unerfülltem Kinderwunsch", , 18 July 2010 (2010-07-18), XP002612708, Retrieved from the Internet: URL:http://www.kinderwunsch.at/blog/catego ry/zech-selektor/ [retrieved on 2010-12-06]

## Description

The present invention relates to a method for producing an enriched DNA strand break-free spermatozoa sample, in particular human enriched DNA strand break-free spermatozoa sample, which can be further used in artificial reproductive technology (i.e. *in vitro* fertilization (IVF), insemination) or diagnostic methods. Accordingly, the present invention relates to a method for producing (an) enriched DNA strand break-free spermatozoa sample(s) from, e.g. seminal fluid (including but not limited to sperm samples/seminal fluid of a poor quality). Said method employs, a selecting device comprising two chambers and a bridge elementas provided herein. In particular, such a method may comprise the following steps: (a) placing a seminal fluid (for example a poor seminal fluid sample that comprises at least 15 % oligo-, at least 32% astheno-, and/or at least 4 % teratozoospermia based on the total amount of the spermatozoa in the seminal fluid) in the first chamber of the selecting device; (b) filling the second chamber of the selecting device with a medium for receiving DNA strand break-free spermatozoa; and (c) connecting both chambers by the bridge element such that a fluid bridge between the first and second chamber occurs. This technique allows the DNA strand break-free spermatozoa to move from the first to the second chamber. The obtained DNA strand break-free spermatozoa sample is of high quality and can be used in various applications. In accordance with the present invention, it was in particular found that the method as provided herein leads in reproductive methods, like in vitro fertilizations, to a surprisingly low rate of abnormalities and/or aneuploidies in the offspring, in particular in the offspring of fathers who are characterized by their low sperm quality. Therefore, the present disclosure relates to means and methods for the reduction of the risk of congenital abnormalities or aneuploidies in artificial reproductive technologies, said means and methods comprising the selection and/or enrichment of spermatozoa in accordance with this invention. In this context, also disclosed are means and methods for the selection and/or enrichment of spermatozoa that have a reduced risk of inducing or leading to congenital abnormalities or aneuploidies after and in the course of artificial reproductive technologies, like in vitro fertilizations.

A relatively high number of patients fail to achieve pregnancy despite the obvious absence of male or female factor of infertility. It is likely that many of these couples actually possess with genomic male factor, including meiotic alterations, aneuploidy or sperm DNA damage. Sperm DNA-fragmentation is increased in poor-quality semen samples and correlates with failed fertilization, impaired pre-implantation development, reduced pregnancy outcome and higher, in particular,malformation rate of the offspring (Zini, A., et al, 2008, "Sperm DNA damage is associated with an increased risk of pregnancy loss after IVF and ICSI: systematic review and meta-analysis, Hum. Reprod. 23, 2663-2668).

In the last years, several tests have been established for analysis of sperm DNA-fragmentation. Amongst other, TdT-mediated-dUTP nick-end labeling (TUNEL), comet assay, sperm chromatin structure assay (SCSA) as well as the sperm chromatin dispersion (SCD) test are those approaches most commonly used in *in vitro* fertilization laboratories. These assays can be subdivided into two categories, those directly detecting DNA damage (e.g. TUNEL) and those measuring DNA fragmentation after a rather mild denaturation process (e.g. SCSA, SCD).

Many factors are thought to induce DNA-fragmentation, such as apoptosis, oxygen radicals, radio- and chemotherapy or environmental toxicants etc.

The DNA damage of the sperm can occur during production and/or transport of the male gametes.

During spermatogenesis screening mechanisms in Sertoli cells are responsible for the induction of apoptosis by marking individual sperms with apoptotic markers which causes phagocytosis of these cells (Billig et al., 1996, "Gondal cell apoptosis: hormone-regulated cell demis", Hum. Reprod. Update 2, 103-117). Testicular endogenous nuclease activity required for facilitating protamination further increases the percentage of DNA damaged spermatozoa (McPherson et al., 1993, "Chromatin structure-function alternations during mammalian spermatogenesis: DNA nicking and repair in elongating spermatides", Eur. J. Histochem. 37, 109-128).

It is being speculated that post-testicular DNA-fragmentation arising during sperm transport through the epididymis is even more frequent since testicular spermatozoa show lower level of DNA damage then epididymal or ejaculated ones.

Furthermore, it is known that immature sperms (e.g. showing cytoplasmatic retentions) produce high levels of reactive oxygen species (ROS), and as sperms are highly packed in the epidiymis, ROS-associated damage of DNA (either directly or via the activation of caspases) is very likely to happen. On average, a 25% increases in seminal ROS level was found to be correlated with a 10% increase in DNA-fragmentation.

However, if testicular and epididymal mechanisms for removal of genomically defective sperm do not work properly a certain percentage of defective germ cells will later end up in ejaculate presenting DNA strand breaks. The influence of such problems on reproductive outcome can only be estimated since it is not known to which degree the oocyte can compensate for a paternal factor and even if a high percentage of sperm in an ejaculate may have fragmented DNA a non-affected sperm could be chosen for artificial insemination. This grey area could be one reason for conventional discussion on the possible effect of sperm DNA strand breaks on further outcome.

One important aspect with respect to sperm DNA fragmentation is the question if DNA strand breaks are single or double since the single defects are properly easier to repair as compared to double-stranded DNA breaks. In this respect it should be kept in mind that the processing of sperm, such as e.g. density gradient centrifugation, could also cause an apparent increase in DNA strand breaks, thus, the sperm processing technique applied for removing DNA-damaged sperm is utmost importance.

Several methods have been developed to enrich DNA strand break-free spermatozoa, for example: Magnetic cell sorting using annexin-V microbeads can effectively separate apoptotic and non-apoptotic spermatozoa (Said et al., 2005, "Advantage of combining magnetic cell speration with sperm preparation techniques", Reprod. Biomed Online 10, 740-746). Other reduced percentage of sperm with DNA strand breaks by either selecting based on maturity, e.g. as assessed by the presence of hyaluronic acid binding-sites on the sperm head (Parmegiani et al., 2010, "Physiologic ICSI: hyaluronic acid (HA) favors selection of spermatozoa without DNA fragmentation and with normal nucleus, resulting in improvement of embryo quality, Fertil. Steril. 93, 598-604) or at higher magnification (Bartoov et al, 2003, "Pregnancy rates higher with intracytoplasmic morphologically selected sperm injection than with conventional intracytoplasmic injection", Fertil. Steril. 80, 1413-1419; Itzkan et al., 2007, "Confocal light adsorption and scattering spectroscopic microscopic monitors organelles in live cells with no exogenous labels", Proc. Natl. Acad.Sci, USA 104, 17255-17260).

As common feature of all these methods, they can only reduce the percentage of sperms revealing DNA damage but never eliminate them from further usage.

Thus, both the actual extent of DNA damage in individual male gametes and the efficiency of certain sperm processing techniques to enrich DNA strand break-free spermatozoa are of clinical importance.

Therefore, there is a high need for a production method which provides spermatozoa samples that are practically free of DNA strand breaks, i.e. there is a need to enrich DNA strand break-free spermatozoa, especially form semen samples of poor quality. Accordingly, there is a need in the art for means and methods for decreasing malformations and/or abnormalities in offspring obtained from artificial reproductive methods, like in vitro fertilizations/inseminations. The enriched DNA strand break-free spermatozoa sample as obtainable by the means and methods provided herein are in particular suitable for further usage, such as in artificial reproductive technology (i.e. IVF, insemination) and for diagnostic purposes.

The present invention is based on the surprising finding that an enriched DNA strand break-free spermatozoa sample can be produced even from seminal fluid of a poor quality. Such a seminal fluid of poor quality is for example, a seminal fluid which comprises at least 15% oligo-, at least 32% astheno-, and/or at least 4% teratozoospermia, based on the total amount of the spermatozoa in the seminal fluid. The herein disclosed method for obtaining a seminal fluid/spermatozoa sample comprising enriched DNA practically free of DNA-strand breaks, may comprise the following steps
(a) placing a seminal fluid (for example of poor quality, like a sperm sample that comprises at least 15% oligo-, at least 32% astheno-, and/or at least 4% teratozoospermia, based on the total amount of the spermatozoa in the seminal fluid) in a first chamber of the selecting device;
(b) filling a second chamber of the selecting device with a medium for receiving DNA strand break-free spermatozoa; and
(c) connecting both chambers by a bridge element such that a fluid bridge between the first and second chamber is formed which allows the DNA strand break-free spermatozoa to move from the first to the second chamber.

The terms "oligo"-, "astheno"- and teratozoospermia", also called "oligoasthenoteratozoospermia" (OAT) refer within the present invention to semen condition that includes, inter alia,
➢ oligozoospermia, i.e. a low number of sperm,
➢ asthenozoospermia, i.e. a poor sperm motility, and
➢ teratozoospermia, i.e. abnormal sperm shape.

These sperm cells properties may be determined by light microscopy using a drop of fresh ejaculate on a Makler chamber to determine the motility and concentration of the sperm cell sample, whereas the morphology is determined using a died sperm smear.

Disorders related to sperm structure or its "morphology" is an important reason of infertility. So far as Kruger criterion if these kind of structural disorders are more than 4 % it is considered of normal.

According to the WHO standard 2010 a generative ejaculate (seminal fluid) comprises at least 15 million sperm cells per millilitre, whereby at least 32% of these sperm cells are progressive motile. The generative ejaculate comprises not more than 15% oligo-, 32% astheno-, and/or 4% teratozoospermia, based on the total amount of spermatozoa.

A non-generative (poor quality) ejaculate (seminal fluid) comprises less than 15 million sperm cells, whereby at least 32 % of the sperm cells are immotile and/or at least 15% of the sperm cells are oligozoospermia, 32% astheno, and/or 4% teratozoospermia, based on the total amount of spermatozoa.

The term "motile" refers according to the present invention to sperm cells which are able to move within a medium, such as a liquid, by their own motion.

In general, the motility of sperm cells is classified in four categories (cf. WHO standard 2010):
(a) Sperm cells which show a fast progressive motility, i.e. these cells are strongest, swim fast in a straight line and are target oriented. Said sperm cells show a velocity of at least 25 micrometer/sec at 37 °C, and at 20 °C a velocity of 20 micrometer/sec,
(b) Sperm cells which show a progressive motility, i.e. these cells also move forward but tend to travel in a curved or crooked motion,
(c) Sperm cells which show non-progressive motility, i.e. these cells have no-progressive motility because they do not move forward despite the fact they move tails. The velocity of these sperm cells is less than 5 micrometer/sec; and
(d) Sperm cells which are immotile, i.e. the cells fail to move.

In a generative ejaculate which may be suitable for the present invention at least 25 % of sperms cells of a sample should show a fast progressive motility (category (a)) or at least 50% of sperm cells of a sample should show a progressive motility (category (a) + (b)). Nevertheless, also an ejaculate in which less than 25 % of the sperm cells of a sample show a fast progressive motility or less than 50% of the sperm cells show a progressive motility may be used in the inventive method.

The motility of the sperm cells may also be determined by microscopy. The motility of the sperm cells is, for example, determined by microscopy using a single drop of fresh ejaculate on a Makler chamber. To more precisely calculate the motility and the speed of spermatozoa, computer programs such as the CASA (Computer Assisted Sperm Analysers) may be used to help diagnose certain male motility related infertilities.

In the inventive method the used seminal fluid preferably may comprise less than 80 million sperm cells, less than 50 million sperm cells, less than 20 million sperm, less than 15 million sperm cells per millilitre. The present invention is preferably carried out with a seminal fluid comprising 5 to below 80 million sperm cells per millilitre.

Furthermore, the seminal fluid may be characterized in that the fluid comprises at least 32 %, at least 35%, at least 40%, at least 45% asthenozoospermia, i.e. the sperm cells show a poor motility or they are immotile (see category (c) and (d) herein above).

Furthermore, the seminal fluid that may be employed as starting material in the inventive method may of poorer quality and may comprise at least 15%, at least 18%, at least 20% oligozoospermia, and/or at least 4%, at least 5%, at least 8% teratozoospermia, based on the total amount of spermatozoa.

The seminal fluid may comprise 32% to 50% astheno-, 15% to 25% oligo-, and/or 4% to 10% teratozoospermia.

According to the present invention the seminal fluid may comprises all three semen condition, i.e. the seminal fluid is a so-called oligoasthenoteratozoospermia, or only one or two of them in any combination.

In the present invention it has been surprisingly found that DNA strand-break free spermatozoa show a high motility in the described selecting device and in the method as provided herein. Thus, in the inventive method, the spermatozoa of higher quality are selected due to their motility/velocity and not due to their morphology (e.g. sperms cells with smaller vacuoles, head deformation and/or cytoplasmic droplets passed the ejaculate-medium barrier).

Therefore, the inventive method allows enrichment of DNA strand break-free spermatozoa from a seminal fluid, in particular of semen/seminal fluid/ solutions comprising sperm cells having a poor quality, like semen/seminal fluid/ solutions comprising sperm cells comprising a high amount of sperm cells that show and/or comprise DNA damages, in particular DNA strand breaks/DNA-fragmentations.

In accordance with the present invention, 5 to 95%, 10 to 90%, 20 to 85%, 25 to 80%, 30 to 50% of the sperm cells in poor quality sperm samples show a DNA-fragmentation determined by the SCD test as described in the paragraph "Material and Methods".

The term "seminal fluid" refers according to the present invention to the ejaculate of a mammalian. In common the ejaculate is obtained by masturbation of mammalian after two to ten days of abstinence, preferably after two to five days of abstinence. For carrying out the method according to the present invention it may be necessary that the seminal fluid contains in addition to the obtained ejaculate a medium suitable for spermatozoa.

In the present invention an ejaculate of a human or animal can be used. In particular in stock-breeding or in animal husbundry it is essential that the used ejaculate for artificial insemination has a high quality. Exemplified species and animals where the means and methods provided herein are of high advantage are cattle, horses, camels, pigs, etc. The present disclosure is in particular useful to avoid and/or reduce the risk for malformations and/or abnormalities in offspring of mammals after artificial reproductive methods. Thus, in order to ensure that the used seminal fluid is of higher quality and avoids or reduces the risk for undesired abnormalitiesthe present method can be used.

It is preferred that the ejaculate/semen/seminal fluid/ solutions comprising sperm cells used in the inventive method is obtained from a human, i.e. a man.

The selecting device which can be used in the inventive method comprises a first and a second chamber as well as a bridge element.

Before the seminal fluid is placed in the first chamber of the selecting device the seminal fluid is not pre-treated, i.e., the sperm cells are not preselected by methods which are commonly used for enrichment of DNA strand break-free spermatozoa e.g. density gradient centrifugation, magnetic cell sorting etc..

It is preferred that the seminal fluid is placed directly in the first chamber of the selecting device after it has been obtained and liquefied from the male or the animal. It is further preferred that the ejaculate/seminal fluid used in the inventive method is not older than 90 minutes, preferably not older than 60 minutes, more preferably not older than 45 minutes, after obtaining the ejaculate, for the mammal, like after ejaculation after masturbation or ejaculation by other means employed, inter alia, in animal husbundry. Preferably, the ejaculate/seminal fluid to be treated in accordance with the present invention is about 1 to about 90 minutes old (time period after the ejaculate/seminal fluid has left the male animal, the male human) before it is placed in the first chamber of the selecting device.

Alternatively, it is also possible to use a frozen/thawed seminal fluid in the inventive method. In that case, preferably directly after masturbation/ejaculation of the mammalian the seminal fluid is frozen for storage and afterwards thawed in order to carry out the inventive method. For freezing, storage and thawing every method which is common in the art may be used. Obviously, when the inventive method is employed sperm samples/seminal fluid etc is to be employed which is thawn and in a corresponding medium, buffer, etc.

After the first chamber is filled, the second chamber is filled with a suitable medium known in the art.

The term "medium" refers to any kind of compound in which the spermatozoa can move. Preferred media are fluid media such as liquids and gels. Said fluid media and gels can also contain further substances such as nutrient solutions, or other influencing substances (c.f. Pentoxyphylin). It is preferred to use a nutrient solution as medium. However, it is evident for the person skilled in the art that the inventive method can also be employed directly on (e.g. non-diluted) seminal fluid/sperm samples.

After filling the second chamber with a medium, the bridge element is mounted on the chambers. The bridge element according to the present invention has an opening in the area of the first chamber and an opening in the area of the second chamber and is mainly or total hollow inside, such that a fluid bridge between the first and second chamber can be formed, whereby the seminal fluid in the first chamber is preferably also covered with a layer of the used medium.

The inventive method is preferably carried out at 37°C. Nevertheless, the inventive method may be carried out at a temperature between 19 to 37 °C.

The DNA strand break-free spermatozoa can travel via said fluid bridge from the seminal fluid of the first chamber to the nutrient solution of the second chamber. Thus, the spermatozoa are not exposed to mechanical load during selection and the DNA strand break-free sperm cells are separated from sperm cells having a DNA damage.

Furthermore, due to the formed fluid bridge a weak electric field occurs between first and second chamber which helps the DNA strand break-free spermatozoa to travel via the fluid bridge, and thus to separate the DNA strand break-free spermatozoa from spermatozoa having a DNA damage.

The obtained enriched DNA strand break-free spermatozoa sample could be removed from the second chamber for example with a pipette for insemination, cryoconservation or diagnostic for extracorporal fertilization. It is also possible to insert one or more egg cells directly into the second chamber for fertilization.

The seminal fluid used in the inventive method comprises 5 to 95 %, 10 to 90%, 15 to 80%, or 20 to 75% DNA strand break-free spermatozoa, based on the total amount of spermatozoa in the seminal fluid, and determined by the SCD test as described in the paragraph "Material and Methods".

The enriched DNA strand break-free spermatozoa sample produced by the method of the invention comprises 70 to 100%, 75 to 98%, or 80 to 95 % of strand break-free spermatozoa, as inter alia determined by the SCD test as described in the paragraph "Material and Methods".

Thus, the inventive method leads to a high concentrated DNA strand break-free concentration (P<0.05) since all selected sperms (100%) showed fast progressive motility.

In the present invention it is preferred that the used selecting device consists of two chambers and the bridge element connects both chambers together such that a fluid bridge between both chambers is formed.

It is further preferred that the seminal fluid of the first chamber and the medium of the second chamber are separated by at least one wall of the chambers which has an upper edge. The bridge element which connects both chambers encloses this upper edge and an upper portion of said wall such that the fluid bridge can be formed in the bridge element.

The fluid bridge is preferably generated by capillary forces.

According to the teaching of the invention, the bridge element becomes saturated complete or partly contacted with the seminal fluid of the first chamber and the medium of the second chamber.

Furthermore, it is preferred that the bridge element has at least one channel formed by limiting walls.

It is further preferred that the bridge element has at least two, more preferably at least three, and even more preferably at least four, most preferably at least ten channels formed by limiting walls. It is preferred that the bridge element has up to 20 channels formed by limiting walls.

It is also preferred that the limiting walls and thus the channel or channels of the bridge element have a U-shaped cross section.

The length of each channel of the bridge element is preferably between 15 to 40 millimetres, more preferably between 20 to 38 millimetres, most preferably between 25 to 35 millimeters, measured from the first to the second chamber. This facilitates the filling of the channel of the bridge element and ensures that the DNA strand break-free spermatozoa are enriched in the second chamber in an optimal way, but the length of each channel is too short to separate the spermatozoa due to the sex characteristics. Thus, the method of the present invention leads specifically to an enriched DNA strand break-free spermatozoa sample.

It is further preferred that at least one channel of the bridge element protrudes farther into the second chamber than into the first chamber.

Moreover, it is preferred that at least one channel of the bridge element is a flat channel. It is more preferred that the bridge element comprises at least two, even more preferred at least five, and most preferred at least at least ten flat channels. According to the present invention the bridge element may comprise up to 20 flat channels.

The term "flat channel" refers according to the present invention to a hollow space having a smaller extension in one dimension than in the other two dimensions. Preferably, the extension of the hollow space in the other two dimensions is a multiple of the extension in the first dimension.

According to the present invention the flat channel has already a defined form, i.e. it has fixed dimensions predefined by the fixed arrangement of the limiting walls. This means that in the filled state, the connection of the chambers by the seminal fluid/medium present in the channel can form in an optimal fashion after the bridge element is mounted on the chambers, and the seminal fluid/medium in the channel suffices, i.e. is not subjected to convection, so that the DNA break-free spermatozoa move through the channel on the basis of their motility. It is thus guaranteed that the selection of the motile spermatozoa is always effected in reproducible, rapid fashion.

Furthermore, it is preferred that the used selecting device is formed so that the first chamber and second chamber are at least partially interconnected by their walls.

In this case the bridge element is mounted on the at least partly interconnected chambers such that the connected walls of the chambers and one side of the bridge element each form a limiting wall of a channel, preferably of a flat channel.

When the bridge element is mounted on the at least partially interconnected chambers, the limiting walls extending from the first to the second chamber have an opening in the area of the first chamber and the second chamber in each case. The so formed channels are fillable with the seminal fluid/medium to generate the fluid bridge between the two at least partially interconnected chambers in which the DNA strand break-free spermatozoa can move from the first to the second chamber. Thus, the seminal fluid in the first chamber is connected with the medium in the second chamber.

Furthermore, it is preferred that the bridge element comprises an adjusting element that each channel is always formed in the same defined fashion when the bridge element is mounted.

Preferably, the distance between the opposing limiting walls forming a channel, or parts of said walls, is predefined such that capillary forces act on the seminal fluid/medium with which the channel is filled, so that upon contact with the medium the channel independently becomes saturated at last partly, preferably completely.

This construction allows that each channel, which is preferably a flat channel, is reliably always formed in predefined, reproducible fashion when the bridge element is mounted on the connecting wall of the chambers by the user, i.e., the channel has the dimensions predefined by the arrangement independently of the handling of the user. The adjusting element makes it possible to give the channel formed an optimal shape for formation of the fluid bridge independently of the handling of the device by the user.

Further preferably, the selecting device used in the method of the present invention is formed such that the opposing limiting walls of the channel have a maximum distance apart of 1.0 millimetre, preferably 0.5 millimetres, more preferably 0.3 millimetres.

Further preferably, the opposing limiting walls forming a channel of the bridge element, or parts of limiting walls, have a minimum distance apart of 0.1 millimetres, preferably 0.2 millimetres. Especially preferably, opposing limiting walls have a distance apart in the range of 0.3 to 0.4 millimetres.

The stated preferred distances between limiting walls ensure an optimal formation preferably of a fluid bridge of seminal fluid, aqueous medium or nutrient solution in the channels through the capillary action of the channels.

It is further preferred that the opposing limiting walls of a channel extend parallel to each other in the selecting device used in the present invention.

Preferably, the channels of the device are formed so that a pair of channels at least partly shares a limiting wall, preferably they share a total limiting wall.

It is further preferred that at least one channel of the used device protrudes with a different distance into the first chamber in comparison with the other channels. Further preferably, all channels of the device protrude with different distances into the first chamber.

The different insertion depths of the channels into the first chamber facilitate the exit of especially a medium with higher surface tension from the channels through improved drop formation, thereby facilitating the filling of the channels. This applies for example to nutrient fluid for spermatozoa, which has a considerably higher surface tension in comparison with water.

Preferably, the first chamber of the used selecting device has annular form and encloses the second chamber. More preferably, the bridge element of the used device is in a rotationally symmetric arrangement.

It is also preferred that the two chambers of the used device are part of a chamber element made of one piece.

The used device can be constructed of materials such as glass or plastic. It is preferred that the selecting device is made of polyethylene.

In the method according to the present invention it is preferred that a selecting device as described in EP 1 432 787 (or WO 94/17742 or WO 03/031564) is used. The disclosure content of EP 1 432 787 as well as of WO 94/17742 and WO 03/031564.

The method of the present invention is carried out preferably in a time period of 20 to 120 minutes, more preferably of 25 to 100 minutes, even more preferably 30 to 60 minutes, most preferably 30 to 40 minutes. This period ensures that the DNA strand break-free spermatozoa are completely separated from the spermatozoa having DNA-fragmentation.

Preferably, the methods disclosed herein are carried out under sterile conditions.

It is further preferred that the seminal fluid is used in an amount of about 1 to about 6 millilitres, preferably about 2 to about 5 milliliters in the inventive method.

It is further preferred that if the selecting device as described above is made of polyethylene, the used minimum volume of the seminal fluid is about 1 millilitre and, if the selecting device is made of glass, the used minimum volume of the seminal fluid is about 2 millilitres.

If the volume of ejaculate is less than required above and in order to obtain the required volume., the difference may be filled up with a suitable medium of buffer solution, like with BM1 medium, or another medium which is allowable and/or useful for semen preparation in order to obtain the required volume.

The disclosure also relates to means and methods for the reduction of the risk of congenital abnormality or aneuploidy in an artificial reproductive technology comprising the selection and/or enrichment of spermatozoa and for the selection and/or enrichment of spermatozoa that have a reduced risk of inducing or leading to a congenital abnormality or aneuploidy after in vitro fertilization.

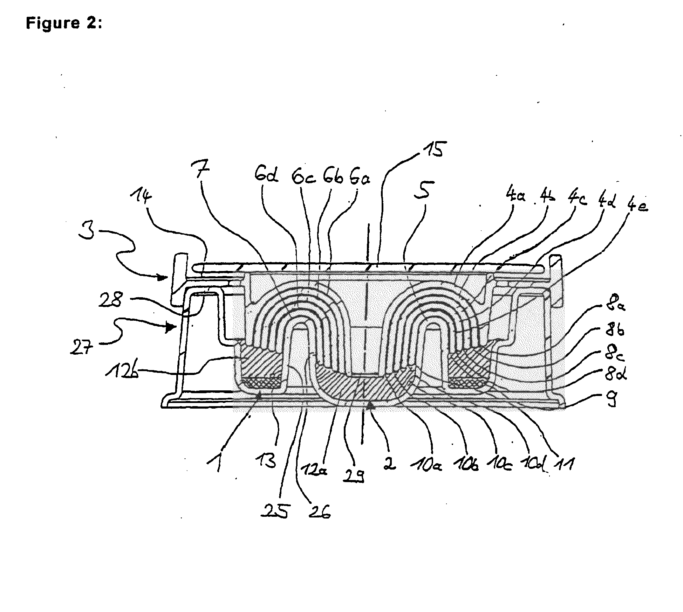

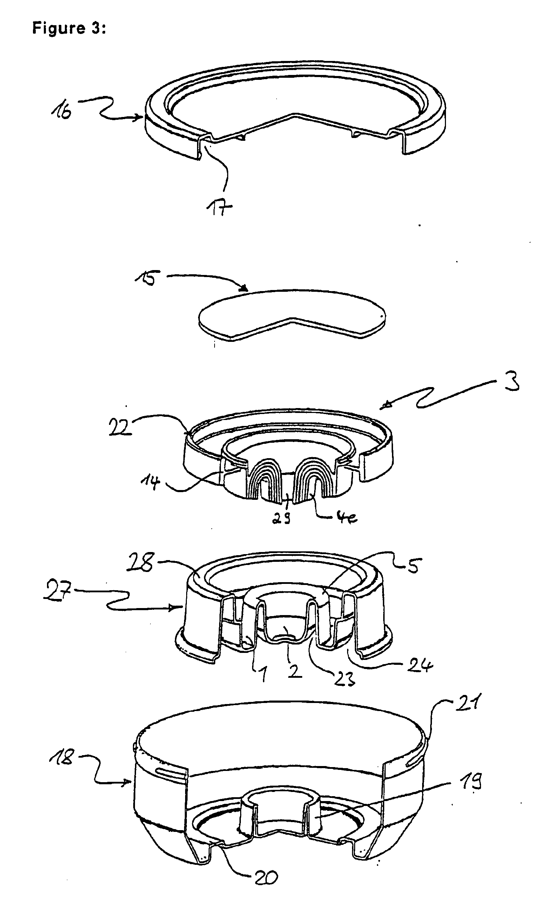

Also disclosed is the use of a device for the selection of spermatozoa from a seminal fluid (or also from spermatozoa provided in other solutions, like buffers etc.) for reducing the risk of a congenital abnormality or aneuploidy in an artificial reproductive technology. Suitable devices for the selection of spermatozoa are described in WO-A1 94/17742 or WO-A2 03/031564. In particular, disclosed is the use of a device for the selection of spermatozoa from a seminal fluid for reducing the risk of a congenital abnormality or aneuploidy in an artificial reproductive technology, wherein the device comprises a first chamber (1) for receiving a seminal fluid medium (12b, 13) containing the spermatozoa to be selected and, separated from it, a second chamber (2) for receiving the selected spermatozoa in a further medium (12a), characterized in that it comprises a bridge element (3) which can be fitted onto the chambers (1, 2) which has at least one flat duct (6a-d) formed by boundary walls (4a-e) which, when the bridge element (3) is fitted, extends from the first chamber (1) to the second chamber (2), has in each case an opening in the region of the first chamber (8a-d) and the second chamber (10a-d) and can be filled with a medium in which the spermatozoa can move such that the medium in the first chamber (12b, 13) is connected to that in the second chamber (12a). An illustrative device to be employed in accordance with the present invention is also shown in the appended figures, like, e.g. figure 2 and figure 3.

Also disclosed is the use of such a device for the selection of spermatozoa that have a reduced risk of inducing or leading to a congenital abnormality or aneuploidy for an artificial reproductive technology. Particularly the device comprises a first chamber (1) for receiving a medium (12b, 13) containing the spermatozoa to be selected and, separated from it, a second chamber (2) for receiving the selected spermatozoa in a further medium (12a), characterized in that it comprises a bridge element (3) which can be fitted onto the chambers (1, 2) which has at least one flat duct (6a-d) formed by boundary walls (4a-e) which, when the bridge element (3) is fitted, extends from the first chamber (1) to the second chamber (2), has in each case an opening in the region of the first chamber (8a-d) and the second chamber (10a-d) and can be filled with a medium in which the spermatozoa can move such that the medium in the first chamber (12b, 13) is connected to that in the second chamber (12a).

Corresponding suitable devices are also illustrated in the appended Fig. 2 and 3.

In a preferred embodiment of the device, the walls of the first chamber (1) and the second chamber (2) are at least partially connected (5) to one another and the bridge element (3) can be fitted onto the chambers (1, 2) such that the connected walls (5) and one side of the bridge element (4e) each form a boundary wall of a flat duct (7) which has an opening in each case in the region of the first chamber (9) and the second chamber (11) and can be filled with a medium in which the spermatozoa can move such that the medium in the first chamber (12b, 13) is connected to that in the second chamber (12a). In this context it is preferred that at least one adjusting element (14) is provided such that the duct (7) formed by the connected walls of the chambers (5) and one side of the bridge element (4e) as boundary walls is always formed in the same, defined, manner when the bridge element (3) is fitted.

It is further preferred that the distance between the opposite boundary walls (4a-e, 5) or parts of the boundary walls (4a-e, 5) forming a duct is predefined such that capillary forces act on the medium with which the duct is filled, with the result that the duct automatically fully or partially soaks itself upon contact with the medium.

In one particular embodiment, the maximum distance between the opposite boundary walls (4a-e, 5) or parts of boundary walls (4a-e, 5) forming a duct (6a-d, 7) is at most 1.0 mm preferably 0.5 mm, and the minimum distance at least 0.1 mm, preferably 0.2 mm.

The opposite boundary walls (4a-e, 5) of a duct (6ad, 7) may preferably in each case run parallel to each other. It is preferred that two ducts (6a-d, 7) each have, at least partially, a common boundary wall (4b-e). The boundary walls (4a-e, 5) of the ducts (6a-d, 7) preferably have a U-shaped cross-section. In a particular embodiment one of the chambers (1, 2) is developed annular (1) and encloses the other chamber (2).

It was surprisingly found in accordance with the present invention that the herein disclosed method for producing (an) enriched DNA strand break-free spermatozoa sample leads to a surprisingly high quantity of spermatozoa for e.g. in vitro fertilization. Surprisingly, this method provides for and leads to the selection and/or enrichment of spermatozoa that have a reduced risk of inducing and/or leading to a congenital abnormality or aneuploidy after in vitro fertilization. Accordingly, the present disclosure pertains for the reduction of the risk of congenital abnormality or aneuploidy in an artificial reproductive technology comprising the selection and/or enrichment of spermatozoa and for the selection and/or enrichment of spermatozoa that have a reduced risk of inducing or leading to a congenital abnormality or aneuploidy after in vitro fertilization.

The disclosure further relates to a method for reducing the risk of congenital abnormality or aneuploidy in an artificial reproductive technology comprising the selection and/or enrichment of spermatozoa, the method comprising the steps of
(a) placing a seminal fluid comprising the spermatozoa in a first chamber of a selecting device;
(b) filing a second chamber of the selecting device with a medium for receiving the selected spermatozoa; and
(c) connecting both chambers by a bridge element such that a fluid bridge between the first and second chamber is formed which allows the spermatozoa from the first to the second chamber.

Further, the invention relates to a method for the selection and/or enrichment of spermatozoa that have a reduced risk of inducing or leading to a congenital abnormality or aneuploidy after in vitro fertilization, the method comprising the steps of
(a) placing a seminal fluid comprising the spermatozoa in a first chamber of a selecting device;
(b) filing a second chamber of the selecting device with a medium for receiving the selected spermatozoa; and
(c) connecting both chambers by a bridge element such that a fluid bridge between the first and second chamber is formed which allows the spermatozoa from the first to the second chamber.

These methods are preferably performed using the above described devices for the selection of spermatozoa.

In the herein disclosed means and methods for the reduction of the risk of congenital abnormality or aneuploidy in an artificial reproductive technology, like in vitro fertilization, and in means and methods for the selection and/or enrichment of spermatozoa having a reduced risk of inducing or leading to such abnormalities or undesired chromosomal damages, the embodiments disclosed herein above in context of the production of (an) enriched DNA strand break-free spermatozoa sample(s) apply here mutatis mutantis.

In particular, these methods are also characterized in that the seminal fluid of the first chamber and the medium of the second chamber are separated by at least one wall of the chambers having an upper edge and wherein the bridge element which connects both chambers encloses the upper edge and an upper portion of said wall.

In addition, the fluid bridge between both chambers may be generated by capillary forces, so that the bridge element becomes saturated complete or partial contact with the seminal fluid of the first chamber and the medium of the second chamber. Said bridge element may have a least one channel formed by limiting walls which extend from the first chamber to the second chamber of the selecting device, whereby said limiting walls of the channel may have, in one embodiment, an U-shaped cross section. As pointed out herein above, said at least one channel of the bridge element may have a length of 15 to 40 millimetres measured from the first to the second chamber and said at least one channel of the bridge element may protrude farther into the second chamber than into the first chamber. In addition, said at least one channel of the bridge element may be a flat channel formed by limiting walls. In an alternative, the means and methods provided herein may be, characterized in that
(a) the walls of the first and second chamber are at least partly interconnected;
(b) the bridge element is mounted on the chambers such that the connected walls of the chambers and one side of the bridge element each form a limiting wall of the channel; and
(c) at least one adjusting element is provided such that the channel is always formed in the same, defined fashion when the bridge element is mounted.

The distance between the opposing limiting walls forming a channel, or parts of said limiting walls, may be predefined such that the capillary forces act on the medium with which is the channel filled, so that the channel independently becomes saturated completely or partly by contact with the medium. The first chamber(s) may be of annular form and may enclose the second chamber.

As discussed herein and as illustrated in the appended example, the present disclosure provides for means and methods for the selection of spermatozoa from (a) seminal fluid(s) in particular for reducing the risk of congenital abnormality or of aneuploidy. The means and methods as provided herein may be carried out such that the produced/obtained enriched DNA strand break-free spermatozoa sample comprises 70 to 100% of DNA strand break-free spermatozoa based on the total amount of the spermatozoa in the sample and as, inter alia, determinated in accordance with to the SCD test. By way of reference to an example, it could be shown in accordance with the present invention that in a human seminal fluid sample that comprised about 80% of fragmented DNA/DNA comprising strand breaks after ejaculation, the amount of fragmented DNA/DNA comprising strand breaks could be reduced to about 2% employing the herein disclosed methods. Such an enrichment of strand-break free DNA could not be obtained by methods known in the art. The methods of the present invention, i.e. the selection of high quality spermatozoa without or with considerably less DNA strand breaks from seminal fluid and the enrichment of DNA strand break-free spermatozoa samples and corresponding methods for the reduction of the risk of congenital abnormalities or aneuploidies in artificial reproductive technologies, may be carried out in short periods of time, e.g. in a time period of about 20 to about 120 minutes.

In the context of the above described uses, means and methods, the artificial reproductive technology is preferably in vitro fertilization (IVF) and/or insemination. In one embodiment, said in vitro fertilization is to be carried out on human patients.

The means and methods provided herein are not limited to human patients but can easily be adapted by the person skilled in the art to other subjects and in life stock, in particular other mammals, like cattle, horses, pigs, camels, etc.

Relevant congenital abnormalities which can successfully reduced by employing the means and methods of the present invention include limb abnormality (dysmelia), congenital abnormality of the heart, congenital abnormality of the nervous system, and congenital abnormality of the gastrointestinal system.

Congenital abnormalities of the limb are for example amelia, ectrodactyly, phocomelia, polymelia, polydactyly, syndactyly, polysyndactyly, oligodactyly, brachydactyly, achondroplasia, congenital aplasia or hypoplasia, amniotic band syndrome, and cleidocranial dysostosis. Congenital abnormalities of the heart are for example patent ductus arteriosus, atrial septal defect, ventricular septal defect, and tetralogy of fallot. Congenital abnormalities of the nervous system are for instance neural tube defects such as spina bifida, meningocele, meningomyelocele, encephalocele, anencephaly, Arnold-Chiari malformation, the Dandy-Walker malformation, hydrocephalus, microencephaly, megencephaly, lissencephaly, polymicrogyria, holoprosencephaly, and agenesis of the corpus callosum. Congenital abnormalities of the gastrointestinal system are for example stenosis, atresia and imperforate.

Due to the ease handling of the described method it is not necessary that the inventive method is carried out by laboratory staff, the method can be carried out by everyone.

Furthermore, in the present invention it is demonstrated that the use of a selecting device as described above leads to an enriched DNA strand break-free spermatozoa sample obtained from a seminal fluid which shows poor quality.

The present invention also relates to the following figures and non-limiting examples.

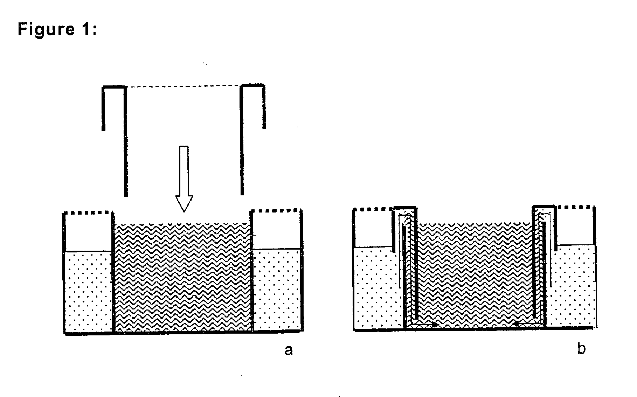

**Figure 1:** Schematic cross-section of the selecting device used in the present invention indicating the ejaculate in the first chamber and medium in the second chamber (wave lines). Cover glass is not shown.
   a. A selecting device consisting of the two chambers (glass or polyethylene) and a bridge element (top left).
   b. After filling of the chambers the bridge element is inserted and creates a fluid bridge allowing motile spermatozoa to swim to the second chamber (theoretical path of migration indicated by arrow).
**Figure 2:** shows a cross section of an exemplified device in the defragmented condition to be employed in the methods of the present invention.
**Figure 3** shows a partial cut-away isometric view of the device according to the invention as well as a container thereof.

### Example I: Production of enriched DNA strand break-free spermatozoa and selection of DNA strand break-free from seminal fluid

### Material and Methods

During the study period 37 patients with known male subfertility who presented at our andrology laboratory for a second analysis of their ejaculate were recruited. The mean age of the men was 37.7±6.5 years. The time of abstinence was recommended with 3-5 days. All ejaculates were processed and analyzed strictly according to the WHO manual (1999).

Half of men suffered from isolated teratozoospermia (51%). A smaller percentage had isolated astheno- (8%) or oligozoospermia (8%). However, the remaining 33% of patients showed a drop in more than one sperm parameter, including 5 cases of oligoasthenoteratozoospermia (OAT) determined by light microscopy.

After control of liquefaction the ejaculate was processed immediately in order to avoid excessive contact between seminal plasma and spermatozoa which could have altered chromatin packaging, thus possibly interfering with DNA-staining.

Three analyses of DNA-fragmentation per patient were made.

### Sample 1

After masturbation, a small volume (ca. 25 µl) of raw semen was kept in order to have a reference value (sample 1). The rest of the ejaculate was split into two unequal parts in order to treat them differently.

### Sample 2

The first volume (1-2 ml) was processed using routine density gradient centrifugation technique (sample 2).

In detail, semen was placed on the top of two layers (40% and 80%) GM501 Gradient (Gynemed, Lensahn, Germany). After layering, the sample was centrifuged at 2000 rpm for 20 minutes at room temperature. Subsequently, both layers containing silane-coated colloidal silica were carefully removed and the pellet resuspended in BM 1 medium (NMS Bio-Medical, Praroman, Switzerland). In order to reduce additional manipulation of the spermatozoa only one centrifugation step was performed at 2000 rpm for 10 minutes at room temperature. Finally, the purified sperm sample was incubated at 37°C and allowed for a swim-up for approximately half an hour until strand break measurement was done.

### Sample 3

In parallel, the first chamber of the sperm selecting device (Zech-Selector, AssTIC Medizintechnik GmbH, Leutasch, Austria, EP 1 432 787) made of glass or polyethylene was filled with 1-3 ml of ejaculate (sample 3).

These chambers accumulate an adequate number of motile sperm without exposure to centrifugation stress. The method was carried out at 37 °C.

After one hour a 25 µl sperm sample was taken from the second chamber and referred to further analysis.

Patients whose ejaculate had to be processed for more than one hour (e.g. due to delayed liquefaction) were excluded from the study for the sake of homogeneity of the study group. Thus, it could be guaranteed that all three samples (neat semen, density gradient and sperm selecting chamber) were analyzed within one hour (including time for liquefaction), in other words prolonged contact with seminal plasma was avoided.

If the volume of ejaculate was large (>5 ml) both types of chambers (glass and polyethylene) were used giving four values in these patients.

In the study it was ensured that a minimum volume of 2 ml was used for the polyethylene chamber and at least 3 ml were filled in the glass chamber. If volume was less than required the difference was filled up with BM 1 medium.

### SCD test

An improved SCD test, the so-called Halosperm® assay (Halotech DNA SL, Tres Cantos, Spain), has been used for the determination of the actual percentage of DNA-damaged sperms. Although the SCD-test has been explained in detail elsewhere (Fernandez et al., 2003, "The sperm chromatin dispersion test: simple method for determination of sperm DNA fragmentation", J. Androl. 24, 59 to 66) a short summary should be given here.

All 3 samples per patient were treated equally. Therefore, a volume of 25 µl ejaculate or sperm suspension was mixed with liquid agarose (50 µl). This mix was pipetted onto precoated slide and covered with small coverslips. The slides were placed in a refrigerator (4°C) for 5 minutes to allow the agarose to produce a microgel with the sperm cells embedded within. After gelation coverslips were gently removed and the slides immediately immersed in an acid solution (7 minutes). Then, the slides were immersed in 10 ml of a lysing solution (25 minutes) since indirect proof of strand breaks does require denaturation of DNA. After a 5- minute washing step in distilled water the probes were dehydrated in increasing concentrations of ethanol (70%-90%-100%) for 2 minutes each, air-dried, and stored at room temperature. For light microscopy, slides were stained. Last but not least, the slides were briefly washed in distilled water and allowed to dry. Strong staining was preferred to easily visualize the periphery of the dispersed DNA halos.

If possible, a minimum of 500 spermatozoa per sample was scored under the 100x objective of the light microscope. Only sperm heads with a distinct halo have been reported to be without strand breaks. It is important to note that sperms with a rather small halo have to be pooled with halo-negative sperms (presumed DNA-damage). A cut-off value of 18% was found above which a severe impact on further outcome is expected.

### Results

A total of 37 men considered to participate in this prospective evaluation dealing with the efficiency of the Zech-Selector with respect to the reduction of DNA damaged sperms within a given ejaculate.

The time of abstinence varied from 2 to 8 days (although 3-5 days were recommended). This period was neither related to the major sperm parameters nor to the percentage of sperms with fragmented DNA. A mean of 4.4 (±1.6) ml ejaculate was produced. The average concentration of sperms was 76.5 ± 103.8 10⁶/ml and the corresponding percentage of progressive motile sperms (WHO (a) and (b) was found to be 36.6 ± 19.4 %. The factor most affected was sperm morphology with only 7.8 ± 6.9 % normally formed spermatozoa on average.

Sperm processing using the density gradient (Sample 2) completely removed immotile sperms and almost removed WHO (c) sperms (ca. 1-2%). However, it did not select for morphologically normal sperm (P>0.05).

The Zech-selector (Sample 3) performed significantly better in concentrating WHO (a) spermatozoa (P<0.05) since all selected sperms (100%) showed fast progressive motility. No such improvement in terms of sperm morphology was observed (P>0.05).

Part of the ejaculates of 37 men was either transferred to the glass chamber (n=19) or the polyethylene device (n=23). Due to a larger volume 5 ejaculates were processed with both types of chambers. Since filling of the polyethylene selector was unsuccessful in two cases (8.7%), 35 patients could finally be included in the present study.

Percentage of strand-break-positive sperms in raw semen was found to be 15.8 ± 7.8% (range: 5.0 to 42.1 %).

Treatment with density gradient marginally (14.2 ± 7.0 %) reduced percentage of strand-break-positive sperms (range: 2.0 to 30.9 %).

Processing the ejaculate with glass Zech-selector, however, completely eliminated strand breaks positive spermatozoa in 17 of 19 cases (89.5%). Two ejaculates showed a minor percentage of affected sperm after processing (<2.5%). Overall, the mean value of DNA-damage after processing with the glass device was 0.4% ± 1.1 % (range: 0 to 2.5%) and differed significantly from density gradient (P<0.001) and raw semen (P<0.001).

Using polyethylene Zech-selectors in 16 of 21 cases (76.2%) no single sperm with strand breaks was counted after sperm selection. The polyethylene device (0.5 ± 0.9%; range: 0 to 3.0%) was significantly better in terms of strand break reduction as compared to density gradient (P<0.001).

There was no such difference between glass and plastic chambers (P=0.64). All 5 patients that had their ejaculate processed with both types of Zech-selectors did not show any strand break-positive sperm after processing.

Thus, it could be demonstrated that the inventive method leads to a DNA damage free spermatozoa sample in contrast to other known prior art selecting methods. Thus, the inventive method provides a good quality sperm sample without additional manipulation, e.g. centrifugation, and thus, it is very likely that no supplementary (artefactal) strand breaks will occur.

## Claims

1. Method for producing an enriched DNA strand break-free spermatozoa sample comprising the following steps
(a) placing a seminal fluid which comprises at least 15% oligo-, at least 32% astheno-, and/or at least 4% teratozoospermia, based on the total amount of the spermatozoa in the seminal fluid, in a first chamber of a selecting device;
(b) filling a second chamber of the selecting device with a medium for receiving DNA strand break-free spermatozoa; and
(c) connecting both chambers by a bridge element such that a fluid bridge between the first and second chamber is formed which allows the DNA strand break-free spermatozoa to move from the first to the second chamber.

2. The method according to claim 1, **characterized in that** the seminal fluid comprises 5 to 95 % DNA strand break spermatozoa, based on the total amount of spermatozoa in the seminal fluid and determined by the SCD test.

3. The method according to claims 1 or 2, **characterized in that** the seminal fluid of the first chamber and the medium of the second chamber are separated by at least one wall of the chambers having an upper edge and wherein the bridge element which connects both chambers encloses the upper edge and an upper portion of said wall.

4. The method according to any one of the preceding claims, **characterized in that** the fluid bridge between both chambers is generated by capillary forces, so that the bridge element becomes saturated complete or partial contact with the seminal fluid of the first chamber and the medium of the second chamber.

5. The method according to any one of the preceding claims, **characterized in that** the bridge element has a least one channel formed by limiting walls which extend from the first chamber to the second chamber of the selecting device.

6. The method according to any one of the preceding claims, **characterized in that** the distance between the opposing limiting walls forming a channel, or parts of said limiting walls, is predefined such that the capillary forces act on the medium with which is the channel filled, so that the channel independently becomes saturated completely or partly contact with the medium.

7. The method according to any one of the preceding claims, **characterized in that** the first chambers is of annular form and encloses the second chamber.

8. The method according to any one of the preceding claims **characterized in that** the method is carried out in a time period of 20 to 120 minutes.

## Patentansprüche

1. Verfahren zur Herstellung einer angereicherten Probe aus Spermatozoen ohne DNA-Strangbrüche, umfassend die folgenden Schritte:
(a) Platzieren von Samenflüssigkeit, die mindestens 15% Oligospermien, mindestens 32% Asthenospermien und/oder mindestens 4% Teratozoospermien, basierend auf der Gesamtmenge der Spermatozoen in der Samenflüssigkeit, umfasst, in eine erste Kammer einer Selektionsvorrichtung;
(b) Füllen einer zweiten Kammer der Selektionsvorrichtung mit einem Medium zur Aufnahme der Spermatozoen ohne DNA-Strangbrüche; und
(c) Verbinden beider Kammern durch ein Brückenelement, so dass sich zwischen der ersten und zweiten Kammer eine Flüssigkeitsbrücke bildet, die es ermöglicht, dass sich die Spermatozoen ohne DNA-Strangbrüche von der ersten in die zweite Kammer bewegen.

2. Verfahren nach Anspruch 1, **dadurch gekennzeichnet, dass** die Samenflüssigkeit 5 bis 95% Spermatozoen mit DNA-Strangbrüchen umfasst, basierend auf der Gesamtmenge der Spermatozoen in der Samenflüssigkeit und durch den SCD-Test bestimmt.

3. Verfahren nach Anspruch 1 oder 2, **dadurch gekennzeichnet, dass** die Samenflüssigkeit der ersten Kammer und das Medium der zweiten Kammer durch mindestens eine Wand der Kammern getrennt sind, die einen oberen Rand haben, und wobei das Brückenelement, das die beiden Kammern verbindet, den oberen Rand und einen oberen Teil der Wand umschließt.

4. Verfahren nach einem der vorstehenden Ansprüche, **dadurch gekennzeichnet, dass** die Flüssigkeitsbrücke zwischen beiden Kammern von Kapillarkräften erzeugt wird, so dass das Brückenelement bei Kontakt mit der Samenflüssigkeit der ersten Kammer und dem Medium der zweiten Kammer vollständig oder teilweise gesättigt wird.

5. Verfahren nach einem der vorstehenden Ansprüche, **dadurch gekennzeichnet, dass** das Brückenelement mindestens einen Kanal hat, der durch die begrenzenden Wände gebildet wird, die sich von der ersten Kammer zu der zweiten Kammer der Selektionsvorrichtung erstrecken.

6. Verfahren nach einem der vorstehenden Ansprüche, **dadurch gekennzeichnet, dass** die Entfernung zwischen den gegenüberliegenden begrenzenden Wänden, die einen Kanal bilden, oder Teilen dieser begrenzenden Wände, vordefiniert ist, so dass die Kapillarkräfte auf das Medium wirken, mit dem der Kanal gefüllt ist, so dass der Kanal bei Kontakt mit dem Medium unabhängig vollständig oder teilweise gesättigt wird.

7. Verfahren nach einem der vorstehenden Ansprüche, **dadurch gekennzeichnet, dass** die erste Kammer ringförmig ist und die zweite Kammer umschließt.

8. Verfahren nach einem der vorstehenden Ansprüche, **dadurch gekennzeichnet, dass** das Verfahren in einer Zeitspanne von 20 bis 120 Minuten durchgeführt wird.

## Revendications

1. Méthode pour produire un échantillon enrichi de spermatozoïdes sans cassure de brin d'ADN comprenant les étapes suivantes :
(a) l'introduction d'un liquide séminal qui comprend au moins 15 % d'oligospermie, au moins 32 % d'asthénospermie, et/ou au moins 4 % de tératozoospermie, sur la base de la quantité totale des spermatozoïdes dans le liquide séminal, dans une première chambre d'un dispositif de sélection ;
(b) le remplissage d'une seconde chambre du dispositif de sélection avec un milieu destiné à recevoir les spermatozoïdes sans cassure de brin d'ADN ; et
(c) le raccordement des deux chambres par un élément de pont de façon à ce qu'un pont fluidique entre la première et la seconde chambre soit formé qui permette aux spermatozoïdes sans cassure de brin d'ADN de se déplacer de la première à la seconde chambre.

2. Méthode selon la revendication 1, **caractérisée en ce que** le liquide séminal comprend de 5 à 95 % de spermatozoïdes avec cassure de brin d'ADN, sur la base de la quantité totale des spermatozoïdes dans le liquide séminal et comme déterminé par le test SCD.

3. Méthode selon les revendications 1 ou 2, **caractérisée en ce que** le liquide séminal de la première chambre et le milieu de la seconde chambre sont séparés par au moins une paroi des chambres comportant un bord supérieur et dans laquelle l'élément de pont qui raccorde les deux chambres englobe le bord supérieur et une partie supérieure de ladite paroi.

4. Méthode selon l'une quelconque des revendications précédentes, **caractérisée en ce que** le pont fluidique entre les deux chambres est généré par des forces capillaires, pour que l'élément de pont se sature complètement ou partiellement au contact avec le liquide séminal de la première chambre et le milieu de la seconde chambre.

5. Méthode selon l'une quelconque des revendications précédentes, **caractérisée en ce que** l'élément de pont comporte au moins un canal formé par des parois limitantes qui s'étendent depuis la première chambre jusqu'à la seconde chambre du dispositif de sélection.

6. Méthode selon l'une quelconque des revendications précédentes, **caractérisée en ce que** la distance entre les parois limitantes opposées formant un canal, ou des parties desdites parois limitantes, est prédéfinie de façon à ce que les forces capillaires agissent sur le milieu avec lequel le canal est rempli, pour que le canal, indépendamment, se sature complètement ou partiellement au contact avec le milieu.

7. Méthode selon l'une quelconque des revendications précédentes, **caractérisée en ce que** la première chambre est de forme annulaire et encercle la seconde chambre.

8. Méthode selon l'une quelconque des revendications précédentes, **caractérisée en ce que** la méthode est mise en oeuvre pendant une période de temps de 20 à 120 minutes.
